# EUROPÄISCHE PATENTANMELDUNG

(11) **EP 4 438 064 A1**
(43) Veröffentlichungstag der Anmeldung: **02.10.2024**
(21) Anmeldenummer: 23166182.8
(22) Anmeldetag: 31.03.2023
(51) Int. Cl.: A61L 2/18, A61L 2/22

(54) **DEKONTAMINATIONSVERFAHREN, INSBESONDERE FÜR PHARMATECHNISCHE ANLAGEN UND ANWENDUNGEN SOWIE DEKONTAMINATIONSANORDNUNG FÜR PHARMATECHNISCHE ANWENDUNGEN**

(71) Anmelder: OPTIMA pharma containment GmbH, 78315 Radolfzell (DE)
(72) Erfinder: KNELLWOLF, Stefan, 9403 Goldach (CH); KAßNER, Thomas, 78315 Radolfzell (DE); DEMMLER, Andreas, 78467 Konstanz (CH); BERNHARD, Lydia, 78333 Stockach (CH)
(74) Vertreter: Patent- und Rechtsanwälte Behrmann Wagner PartG mbB

(57) **Zusammenfassung**

Die Erfindung betrifft ein Dekontaminationsverfahren, insbesondere für pharmatechnische Anwendungen,
gekennzeichnet durch die Verfahrensschritte: Einbringen von einem Dekontaminationsmittel in wenigstens eine Gasleitung (07), die zur Versorgung von wenigstens einem, in einem Isolatorraum (02) angeordneten, bevorzugt als Zweistoffdüse ausgebildeten, Zerstäuber (04) mit einem Trägergasstroms, bevorzugt Druckluft, wobei zum Erzeugen eines Dekontaminationsaerosols in einem Dekontaminationsbetrieb des Isolatorraums (02) der wenigstens eine Zerstäuber (04) über mittels elektronische Steuermittel ansteuerbare Ventilmittel mit Dekontaminationsflüssigkeit, bevorzugt Wasserstoffperoxid, aus einem Dekontaminationsmittelvorratsbehälter (06) versorgt wird, wobei der wenigstens eine Zerstäuber (04) gasleitend mit einer Gasdruckquelle (08), über die Gasleitung (07) verbunden ist und in der Gasleitung (07) zwischen der Gasdruckquelle (08) und den Zerstäubern (04), bevorzugt individuell für jeden Zerstäuber (04), ein Filter (11) zur Filterung des Trägergasstroms angeordnet ist.

## Beschreibung

Die vorliegende Erfindung betrifft ein Dekontaminationsverfahren, insbesondere für pharmatechnische Anwendungen und Anlagen, nach dem Oberbegriff des Anspruchs 1. Ferner betrifft die vorliegende Erfindung eine Dekontaminationsanordnung, insbesondere für pharmatechnische Anwendungen, nach dem Oberbegriff des Anspruchs 12.

Im Stand der Technik sind bereits verschiedene Dekontaminationsanordnungen und Vorrichtungen bekannt, um einen Isolatorraum, vorzugsweise aufweisend einen Plenumraum und einen gasleitend mit dem Plenumraum verbundenen Manipulatorraum, zu dekontaminieren. Vorrichtungen mit einem entsprechenden Isolatorraum, beispielsweise ausgebildet als Isolator, kommen auch aber keinesfalls ausschließlich beim Abfüllen, Verpacken, Umverpacken, und Behandeln von Pharmazeutika und/oder Medizintechnikprodukten zum Einsatz. Die Dekontamination dient dem Zweck eine keimfreie und/oder sterile Umgebung herzustellen, um eine Kontaminierung der im Betrieb des Isolatorraums darin angeordneten Stoffe und Produkte zu verhindern.

Aus dem Stand der Technik sind zur Dekontamination des Isolatorraums unterschiedliche Ansätze bekannt. Beispielsweise wird in der EP 2 719 962 A1 ein Wasserstoffperoxid-Verdampfer gelehrt. Die US 6,010,400 A sieht ebenfalls ein Wasserstoffperoxid-Verdampfer vor.

Aus dem Stand der Technik sind jedoch auch andere Ansätze bekannt, die beispielsweise auf der Grundlage von Zerstäubern, beispielsweise Zweistoffdüsen, aus einer Dekontaminationsflüssigkeit und einem Trägergas oder Treibgas ein Dekontaminationsaerosol herstellen und im Isolatorraum, bevorzugt im Plenumraum und Manipulatorraum, verteilen. Als Dekontaminationsmittel können bevorzugt ebenfalls flüssiges Wasserstoffperoxidlösungen zum Einsatz kommen. Ein derartiger Ansatz wird beispielsweise in der EP 3 409 300 A1 verfolgt, wobei dort zusätzlich zur Verwendung von Zerstäubern, insbesondere Zweistoffdüsen, das Dekontaminationsmittel, insbesondere die Dekontaminationsflüssigkeit über eine mit den Zerstäubern verbundene Ringleitung bereitgestellt wird.

Die Herstellung des Dekontaminationsaerosols aus Dekontaminationsmitteln, insbesondere Dekontaminationsflüssigkeit und Trägergas, beispielsweise Druckluft, soll grundsätzlich dazu dienen etwaige Dekontaminationen im Isolatorraum zu verhindern oder zu beseitigen.

Es besteht jedoch auch die Gefahr, dass über das Trägergas oder den Trägergasstrom, beispielsweise Druckluft, auch oder trotz entsprechender Filtermechanismen Dekontaminationen in den Isolatorraum gelangen. Auch die Gasleitungen und Filter der Gasleitungen für den Trägergasstrom selbst sind potentielle Quellen für Verunreinigungen und Kontaminationen, die dann durch den Trägergasstrom entsprechend in den Isolatorraum eingebracht werden können. Dabei ist besonders kritisch, dass in bestimmten Situationen der Dekontamination des Isolatorraums die Zerstäuber, insbesondere Zweistoffdüsen, auch noch Trägergasstrom ausstoßen oder in den Isolatorraum liefern, wenn kein Dekontaminationsmittel oder keine Dekontaminationsflüssigkeit mehr zerstäubt oder gegeben wird. Dieser Nachlauf des Trägergasstroms, beispielsweise von einer Gasdruckquelle zu den Zerstäubern und damit durch die entsprechenden Gasleitungen und/oder Filter, dient beispielsweise zum Testen der Flussrate des Trägergasstroms sowie zum Testen der Dichtigkeit des Systems.

Gerade in solchen Situationen, in denen der Trägergasstrom weiterhin von der Gasdruckquelle über die Gasleitung und/oder Filter in den Isolatorraum eingebracht wird, ohne dass gleichzeitig Dekontaminationsmittel, insbesondere Dekontaminationsflüssigkeit über die Zerstäuber ausgegeben wird, besteht das Risiko, dass Verunreinigungen oder Kontaminierungen in den Isolatorraum gelangen und von dort in ein Produkt oder ein Mittel gelangen, welches in dem Isolatorraum gehandhabt, beispielsweise abgefüllt, verpackt, umverpackt oder dosiert wird..

Vor diesem Hintergrund besteht die Aufgabe der vorliegenden Erfindung darin bekannte Dekontaminationsanordnungen und Dekontaminationsverfahren, insbesondere für pharmatechnische Anwendungen, zur Dekontamination eines Isolatorraums so weiter zu entwickeln, dass die Nachteile im Stand der Technik überwunden und insbesondere ausgeschlossen wird, dass über den durch eine Gasleitung geförderten Trägergasstrom, der zur Versorgung von Zerstäubern und zur Herstellung eines Dekontaminationsaerosols dient, Dekontaminierungen in den Isolatorraum eingebracht werden.

Die vorgenannte Aufgabe wird im Hinblick auf ein Dekontaminationsverfahren mit den Merkmalen des Anspruchs 1 gelöst. Bezüglich einer Dekontaminationsanordnung wird die oben genannte Aufgabe mit den Merkmalen des Anspruchs 12 gelöst.

Vorteilhafte Ausführungen der Erfindung sind Gegenstand der nachfolgenden Beschreibung, Figurenbeschreibung, der Figuren sowie der Unteransprüche.

Grundsätzlich sollen nachfolgend verfahrensmäßig offenbarte Merkmale auch als entsprechend verfahrensmäßig offenbart und beanspruchbar gelten, sowie umgekehrt.

Im Hinblick auf das Dekontaminationsverfahren, insbesondere für pharmatechnische Anwendungen, ist im Sinne der vorliegenden Erfindung vorgesehen, dass ein Einbringen von Dekontaminationsmittel in wenigstens eine Gasleitung, die zur Versorgung von wenigstens einem, in einem Isolatorraum angeordneten, bevorzugt als Zweistoffdüse ausgebildeten Zerstäuber mit einem Trägergasstrom, bevorzugt Druckluft, dient, erfolgt, wobei zum Erzeugen eines Dekontaminationsaerosols in einem Dekontaminationsbetrieb des Isolatorraums der wenigstens eine Zerstäuber über mittels elektrische Steuermittel ansteuerbare Ventilmittel mit Dekontaminationsmittel, bevorzugt Wasserstoffperoxid, aus einem Dekontaminationsmittelvorratsbehälter versorgt wird und wobei ferner der wenigstens eine Zerstäuber gasleitend mit einer Gasdruckquelle über die Gasleitung verbunden ist und in der Gasleitung zwischen der Gasdruckquelle und dem Zerstäuber, bevorzugt individuell für jeden Zerstäuber, ein Filter zur Filterung des Trägergasstroms angeordnet ist.

Das erfindungsgemäße Verfahren beruht damit auf dem Grundgedanken Dekontaminationsmittel, zumindest in gewissen Dekontaminationsintervallen auch in die Gasleitung einzubringen, die ansonsten nur zur Leitung des Trägergasstroms genutzt wird, um den Zerstäuber, bevorzugt die Zweistoffdüse, mit dem Trägergas oder Treibgas zu versorgen, um aus einem Dekontaminationsmittel, bevorzugt einer Dekontaminationsflüssigkeit ein Dekontaminationsaerosol zu erzeugen oder zu zerstäuben.

Dadurch können, wie nachgehend im Detail weiter ausgeführt werden wird, die Gasleitung und/oder die Filter innerhalb der Gasleitung entsprechend dekontaminiert werden, sodass nachfolgender Trägergasstrom, beispielsweise Druckluft, nicht dadurch verunreinigt oder kontaminiert werden kann, dass innerhalb der Gasleitung und/oder Filter Kontaminationen vorliegen.

Mit anderen Worten ausgedrückt, wird die Gasleitung zur Bereitstellung des Trägergasstroms der Zerstäuber kurzzeitig zweckentfremdet und zur Führung von Dekontaminationsmittel genutzt oder eingesetzt, um die Gasleitung selbst oder das Innere der Gasleitung und/oder Filter oder Teile von Filtern innerhalb der Gasleitung zu dekontaminieren. Die Dekontamination der Gasleitung(en) kann vorteilhaft vor und/oder nach der Dekontamination des Isolatorraums durchgeführt werden. Besonders vorteilhaft kann zusammen, insbesondere vor, der Dekontamination der Gasleitungen auch ein Tausch von Filtern der Gasleitungen durchgeführt werden.

Bezüglich des grundsätzlichen Dekontaminationsverfahrens zur Dekontaminierung des Isolatorraums kann grundsätzlich auf die EP 3 409 300 A1 verwiesen werden, deren Inhalt bezüglich des Aufbaus der Dekontaminationsanordnung und des Ablaufs des Dekontaminationsverfahrens durch Verweis in die vorliegende Offenbarung miteinbezogen wird.

Die Anwendung des erfindungsgemäßen Dekontaminationsverfahrens und der erfindungsgemäßen Dekontaminationsanordnung sind aber nicht auf die in der oben genannten Beschreibung offenbarten Vorrichtungen und Verfahren beschränkt. Grundsätzlich kann das erfindungsgemäße Verfahren und die erfindungsgemäße Vorrichtung in jedem Dekontaminationsverfahren und bei jeder Dekontaminationsanordnung zum Einsatz kommen, bei der ein Trägergasstrom von einer Gasdruckquelle erzeugt und über eine Gasleitung an wenigstens einen Zerstäuber geleitet wird, um im Bereich der Zerstäuber oder beim Austritt aus dem Zerstäuber ein Dekontaminationsmittel, beispielsweise flüssiges Wasserstoffperoxid, zu zerstäuben und in ein Dekontaminationsaerosol zu überführen.

In einer ersten vorteilhaften Ausführungsform des Verfahrens kann vorteilhaft sein, dass flüssiges Dekontaminationsmittel, beispielsweise flüssiges H₂O₂, in die Gasleitung eingebracht wird und mit dem Trägergasstrom über den Zerstäuber in den Isolatorraum ausgetragen wird. Diese Ausgestaltung hat mehrere Vorteile. Einerseits kann der sowieso in der Gasleitung geführte Trägergasstrom genutzt werden, um das eingebrachte, flüssige Dekontaminationsmittel auszutragen. Es sind dementsprechend keine zusätzlichen Fördermechanismen oder sonstige Vorkehrungen zu treffen, um das in die Gasleitung eingebrachte, flüssige Dekontaminationsmittel auszutragen. Die Verwendung von flüssigem Dekontaminationsmittel, beispielsweise flüssigem Wasserstoffperoxid, hat den Vorteil, dass damit die zu dekontaminierenden Oberflächen, insbesondere der Gasleitung, besonders gut und effektiv benetzt werden können und so eine effektive Dekontamination der Gasleitung durchgeführt werden kann.

Im Rahmen der vorliegenden Offenbarung wird teilweise von einem Zerstäuber und der damit verbundenen Gasleitung zur Bereitstellung des Trägergasstroms gesprochen. Die soll aber keinesfalls limitierend auf genau einen Zerstäuber wirken. Stattdessen ist regelmäßig bei derartigen Dekontaminationsverfahren und Dekontaminationsanordnungen eine Vielzahl von Zerstäubern vorgesehen, die an unterschiedlichen Orten des Isolatorraums, bevorzugt des Plenumraums und/oder des Manipulatorraums angeordnet sind, um möglichst effektiv und gleichmäßig eine entsprechend Konzentration von Dekontaminationsaerosol in den Räumen herzustellen oder aufzubauen. Grundsätzlich kann es jedoch vorteilhaft sein, wenn das Einbringen des Dekontaminationsmittels, beispielsweise des flüssigen Dekontaminationsmittels, gemäß der vorangehend beschriebenen Ausführungsform, nicht an einem zentralen Ort, beispielsweise unmittelbar hinter der Gasdruckquelle, eingebracht wird, sondern in einem gegenüber der Gasdruckquelle stromabwärts (in Bezug auf die Flussrichtung des Trägergasstroms während der Zerstäubung von Dekontaminationsmittel durch die Zerstäuber) erfolgt, sodass bevorzugt jede einzelne Gasleitung, die mit einem Zerstäuber gasleitend verbunden ist oder jeder Zweig eines entsprechenden Gasleitungssystems einzeln und separat durch das Einbringen von Dekontaminationsmittel dekontaminiert werden kann.

In einer weiteren, vorteilhaften Ausgestaltung kann vorgesehen sein, dass das flüssige Dekontaminationsmittel an einem, bevorzugt für jeden Zerstäuber individuellen, Einleitungspunkt in die Gasleitung eingebracht wird. Die Verwendung eines Einleitungspunktes, bevorzugt zwischen Gasdruckquelle und Zerstäuber stellt sicher, dass das Dekontaminationsmittel, insbesondere das flüssige Dekontaminationsmittel die notwendigen und relevanten Bereiche der Gasleitung, beispielsweise ab einem entsprechenden Filter, durchströmt oder passiert. Wie vorangehend bereits ausgeführt, kann die individuelle Einleitung oder das individuelle Einbringen von flüssigem Dekontaminationsmittel an einem individuell einem Zerstäuber zugeordneten Einleitungspunkt in der Gasleitung sicherstellen, dass jede einzelne, individuell einen Zerstäuber mit Trägergasstrom versorgende Gasleitung ausreichend mit flüssigem Dekontaminationsmittel beaufschlagt und die Gasleitung folglich sicher dekontaminiert wird.

In einer weiteren, besonders vorteilhaften Ausgestaltung des Dekontaminationsverfahrens ist vorgesehen, dass das flüssige Dekontaminationsmittel an einem Einleitungspunkt eingebracht wird, der in der Gasleitung zwischen einem Filter und dem Zerstäuber angeordnet ist.

Dadurch kann sichergestellt werden, dass ab dem Filter und einschließlich einer Seite oder einer Oberfläche des Filters bis zum Zerstäuber und die gesamte dazwischenliegende Gasleitung durch das flüssige Dekontaminationsmittel, bevorzugt das flüssige Wasserstoffperoxid, gereinigt und dekontaminiert wird. Dabei kann gegebenenfalls vorteilhaft die Menge oder das Volumen an flüssigem Dekontaminationsmittel verringert werden, wenn der Abschnitt der Gasleitung zwischen Filter und Zerstäuber nicht allzu groß ist, bevorzugt im Bereich von einigen Metern liegt. Bevorzugt ist der Einleitungspunkt möglichst nah an einer Position des Filters entlang der Gasleitung angeordnet.

In einer weiteren, ebenfalls besonders vorteilhaften Ausführungsform des Verfahrens kann vorgesehen sein, dass zur Dekontamination der Gasleitung ein Behälter mit einem, bevorzugt vordosierten, Volumen an flüssigem Dekontaminationsmittel während einer zeitweisen Verbindung an einer verschließbaren Kupplung der Gasleitung am Einleitungspunkt angekoppelt und während der zeitweisen Verbindung entleert wird. Als Behälter können beispielsweise vorgefertigte oder vorgefüllte Patronen, Kanülen oder Spritzen dienen, die mit einem entsprechenden Dekontaminationsmittel, beispielsweise flüssigem Wasserstoffperoxid befüllt sind und dann über eine verschließbare Kupplung der Gasleitung am Einleitungspunkt angekoppelt werden und während der Verbindung zwischen dem Behälter und der Kupplung die Überführung des flüssigen Dekontaminationsmittels in die Gasleitung stattfindet. Nach dem Abschluss des Einbringens des flüssigen Dekontaminationsmittels in die Gasleitung kann der Behälter über die verschließbare Kupplung wieder vom Einleitungspunkt abgetrennt und die Kupplung verschlossen werden. Bevorzugt wird vor und/oder nach dem Herstellen der Verbindung zwischen der verschließbaren Kupplung und dem Behälter zunächst ein Teil der verschließbaren Kupplung oder die gesamte verschließbare Kupplung dekontaminiert und/oder desinfiziert, um zu verhindern, dass über die Kupplung eine Kontamination der Gasleitung resultiert.

In einer weiteren, besonders vorteilhaften Ausgestaltung des Verfahrens kann vorgesehen sein, dass zur Dekontamination der Gasleitung das flüssige Dekontaminationsmittel über eine permanente Zuleitung bereitgestellt wird, die über eine wenigstens mittelbare Verbindung zwischen dem Dekontaminationsflüssigkeitsbehälter und dem Einleitungspunkt, bevorzugt mittels entsprechender Fördermittel, Dekontaminationsmittel aus dem Dekontaminationsmittelvorratsbehälter in die Gasleitung fördert. Diese Ausgestaltung verursacht zwar wegen der permanenten Leitungen oder Verbindungen zwischen dem Dekontaminationsflüssigkeitsbehälter und den Einleitungspunkten aufseiten der zu dekontaminierenden Gasleitung einen etwas größeren anlagentechnischen Aufwand, gleichzeitig ermöglicht diese Ausführung aber ein weitestgehend vollautomatisches Dekontaminieren der Gasleitung unter besonders vorteilhafter und synergetischer Verwendung des ohnehin für das Dekontaminationsverfahrens des Isolatorraums vorgesehenen Dekontaminationsmittelvorratsbehältes. Mit anderen Worten ausgedrückt bedeutet dies, dass in der vorliegenden Ausführungsform im Dekontaminationsverfahren zur Dekontaminierung der Gasleitung eine zweite Verbindung zwischen dem Dekontaminationsmittelvorratsbehälter und dem Zerstäuber hergestellt wird, wobei die erste Verbindung, beispielsweise die Speisung des Zerstäubers mit Dekontaminationsmittel im Dekontaminationsverfahren des Isolatorraums bereitstellt, bei dem über die Gasleitung ein Trägergasstrom, beispielsweise Druckluft, bereitgestellt wird. Die zweite Verbindung zwischen dem Dekontaminationsmittelvorratsbehälter und dem Zerstäuber verläuft über die wenigstens mittelbare Verbindung zum Einleitungspunkt der Gasleitung, von dort aus durch die Gasleitung und ebenfalls zum Zerstäuber, wobei die bevorzugten Fördermittel dann Dekontaminationsmittel, bevorzugt flüssiges Dekontaminationsmittel bis zum Einleitungspunkt fördern, von wo aus es vorteilhaft durch einen auch ansonsten durch die Gasleitung förderbaren Trägergasstrom ausgetragen, bevorzugt durch den Zerstäuber ausgetragen wird.

In einer weiteren, vorteilhaften Ausführungsform des Verfahrens kann vorgesehen sein, dass das Dekontaminationsmittel in Form eines Dekontaminationsaerosols in die Gasleitung eingebracht wird. Bei der eingangs beschriebenen Technologie hat sich die Verwendung von Dekontaminationsaerosol für die Dekontamination des Isolatorraums bereits sehr bewährt. Gleichermaßen sind in Anlagen und bei Dekontaminationsverfahren, wie eingangs beschrieben, auch entsprechende Mittel vorgesehen und Verfahren etabliert, um Dekontaminationsaerosol zu erzeugen und deren Konzentration zu stabilisieren. Deshalb kann unter synergetischer Ausnutzung dieser Verfahren und Vorrichtungsmerkmale besonders vorteilhaft auch die Dekontamination der Gasleitung, die in dem Zerstäuber mündet über entsprechende Dekontaminationsaerosole bewirkt werden.

In einer vorteilhaften Weiterbildung kann dazu vorgesehen sein, dass das Dekontaminationsaerosol aus dem Isolatorraum in die Gasleitung, bevorzugt durch ein rückwärtsgerichtetes Passieren des Zerstäubers, eingebracht wird und durch einen Trägergasstrom in die Gasleitung eingebracht, insbesondere eingesaugt, wird, der eine umgekehrte Flussrichtung aufweist, als der Trägergasstrom während des Dekontaminationsbetriebs des Isolatorraums. Mit anderen Worten ausgedrückt sieht die vorliegende Ausführungsform in besonders vorteilhafter Weise vor, dass Dekontaminationsaerosol im Isolatorraum, wie es auch bei der Dekontamination des Isolators selbst erzeugt wird, durch eine umgekehrte Beschaltung oder eine umgekehrte Ansteuerung der Gasdruckquelle der mit den über die Gasleitungen verbundenen Zerstäubern aus dem Isolatorraum über die jeweiligen Öffnungen der Zerstäuber zurück in die Gasleitung geführt, insbesondere gesaugt wird. Dazu muss lediglich eine entsprechende Konzentration an Dekontaminationsaerosol in dem Isolatorraum erzeugt und aufrechterhalten werden und die Gasdruckquelle so betrieben werden, dass kein Überdruck in den Gasleitungen entsteht, der einen Trägergasstrom ausgehend von der Gasdruckquelle in Richtung der Zerstäuber bewirkt, sondern dass umgekehrt ein Unterdruck in den Gasleitungen generiert wird, der dafür sorgt, dass ein Trägergasstrom aus dem Isolatorraum über die Zerstäuber und die Gasleitung - und gegebenenfalls durch etwaige Filter in der Gasleitung - in Richtung der Gasdruckquelle strömt.

Wenn die Gasdruckquelle beispielsweise als umschaltbare Pumpe ausgebildet ist, kann das Umkehren des Trägergasstroms einfach und effektiv erfolgen. Die gasförmigen Teile des Dekontaminationsmittels des Dekontaminationsaerosols kann dabei auch Filter, insbesondere hydrophobe Filter in der Gasleitung passieren und jenseits dieser Filter eine Dekontamination der Gasleitung erreichen. Flüssige Bestandteile des Dekontaminationsaerosols können sich in gewünschter und beabsichtigter Weise an den Innenoberflächen der Gasleitung anlagern und/oder kondensieren um dort die Innenoberfläche der Gasleitung zu dekontaminieren. Besonders vorteilhaft ist darauf zu achten, dass der von der Gasdruckquelle erzeugte relative Unterdruck nicht zu groß ausfällt, da sonst innerhalb der Gasleitung die Tröpfchen oder Flüssigkeitspartikel des Dekontaminationsaerosols zu schnell oder gar schlagartig in Form einer Blitzverdampfung in die Gasphase übergehen, wodurch die Dekontaminationswirkung in der Gasleitung begrenzt oder beschränkt wird. Vorteilhaft beträgt der Unterdruck in der Gasleitung zwischen 500 und 950mbar.

Das eingebrachte, insbesondere eingesaugte Dekontaminationsaerosol kann vorteilhaft eine Konzentration des Dekontaminationsmittels von 400 bis 1200 ppm enthalten. Bei einer zu geringen Konzentration von Dekontaminationsmittel in dem Dekontaminationsaerosol kann eine zuverlässige Dekontamination in der Gasleitung nicht mehr gewährleistet werden.

In einer weiteren, besonders vorteilhaften Ausgestaltung des Dekontaminationsverfahrens kann vorgesehen sein, dass ein erster Teil einer Gesamtheit von mehreren Zerstäubern ein Dekontaminationsaerosol in dem Isolatorraum zerstäubt, während ein zweiter Teil der Gesamtheit der mehreren Zerstäuber das Dekontaminationsaerosol aus dem Isolatorraum entnimmt und insbesondere in die Gasleitung (EM) des zweiten Teils der Gesamtheit der Zerstäuber eindringt, insbesondere einsaugt. Es hat sich herausgestellt, dass eine zunächst über ein Teil oder die Gesamtheit der mehreren Zerstäuber hergestelltes Dekontaminationsaerosol in dem Isolatorraum nicht ausreicht, um gleichzeitig oder nacheinander alle Gasleitungen, bevorzugt durch entsprechendes Einsaugen des Dekontaminationsaerosols aus dem Isolatorraum über die Zerstäuber, zu dekontaminieren.

Es ist jedoch gelungen, dass mit einem ersten Teil oder einer ersten Gruppe von Zerstäubern ein Dekontaminationsaerosol aus Dekontaminationsmittel und Trägergas Strom nachgeliefert wird, um eine Mindestkonzentration an Dekontaminationsmittel oder Dekontaminationsaerosol in wenigstens einem Bereich des Isolatorraums aufrechtzuerhalten, um gleichzeitig über einen zweiten Teil der Gesamtheit der mehreren Zerstäuber ein Teil der Dekontaminationsaerosols zu entnehmen, insbesondere einzusaugen und damit die anschließenden oder angrenzenden Gasleitungen zu dekontaminieren.

Bevorzugt wird das Verfahren so durchgeführt, dass die Zerstäuber, die Dekontaminationsaerosol herstellen oder liefern und die Zerstäuber die Dekontaminationsaerosole abführen oder einsaugen in räumlicher Nähe zueinander angeordnet sind. Weiter vorteilhaft kann vorgesehen sein, wenn die entsprechende Dekontamination von Gasleitungen sukzessive oder einzeln nacheinander durchgeführt wird. Alternativ kann auch gruppenweise eine Dekontamination der Gasleitungen vorgesehen sein. Die jeweilige Identifikation von sinnvollen Reihenfolgen und/oder sinnvollen Gruppen von Zerstäubern hängt auch von der Geometrie und dem genauen Aufbau des Isolatorraums und der dortigen Anordnung der Zerstäuber, beispielsweise in einem Plenumraum und einem Behandlungsraum, ab.

In dieser Ausgestaltung kann es besonders vorteilhaft sein, wenn mehrere Gasdruckquellen vorgesehen sind und unabhängig voneinander betrieben werden können oder mir einer Gasdruckquelle gleichzeitig entsprechende Unterdrücke und Überdrücke erzeugbar und aufrechterhaltbar sind, beispielsweise durch eine variable/schaltbare gasleitende Verbindung mit einer Eingangsseite und einer Ausgansseite einer Pumpe.

In einer weiteren, besonders vorteilhaften Ausführungsform des Verfahrens kann vorgesehen sein, dass vor dem Einbringen, insbesondere Einsaugen, des Dekontaminationsaerosols in die Gasleitung die Gesamtheit der mehreren Zerstäuber ein Dekontaminationsaerosol in den Isolatorraum zerstäubt. Dadurch kann eine Startkonzentration schnell und effektiv erreicht werden, bevor dann einzeln oder gruppenweise Zerstäuber und angeschlossene Gasleitungen mit dem Einbringen, insbesondere Einsaugen, von Dekontaminationsaerosol beginnen während andere Teil der Gesamtheit der Zerstäuber Dekontaminationsaerosol nachliefern und damit ein zu starkes oder zu schnelles Absinken der Konzentration an Dekontaminationsmittel oder Dekontaminationsaerosol in dem Isolatrraum verhindern.

In einer weiteren, besonders vorteilhaften Ausführungsform des Verfahrens kann vorgesehen sein, dass nach dem Einbringen, insbesondere Einsaugen des Dekontaminationsaerosols in die Gasleitung die Flussrichtung des Trägergasstroms umgekehrt und in der Gasleitung verbliebenes, bevorzugt flüssiges Dekontaminationsmittel und/oder Dekontaminationsaerosol mittels des Trägerstroms über die Zerstäuber in den Isolatorraum ausgetragen wird. Dies verhindert, dass Rückstände des Dekontaminationsmittels und/oder des Dekontaminationsaerosols in den Gasleitungen verbleiben und dort Ablagerungen und/oder Verunreinigungen ausbilden. Wie oben bereits ausgeführt ist es für die Ausführungsform, die ein Einsaugen von Dekontaminationsaerosol über die Zerstäuber in die Gasleitungen vorsieht ohnehin erforderlich, dass die Richtung des Trägergasstroms durch die Gasleitung umgekehrt werden kann. Dementsprechend kann in einer entsprechenden Weiterbildung besonders vorteilhaft vorgesehen sein, dass nach Abschluss der Dekontamination der Gasleitungen die Richtung des Trägergasstroms erneut umgekehrt wird, um Reste des Dekontaminationsmittels und/oder des Dekontaminationsaerosols wieder aus den Gasleitungen über die Zerstäuber in den Isolatorraum auszutreiben. Dies ist auch deshalb besonders sinnvoll, weil aus der allgemeinen Dekontamination des Isolatorraums entsprechende Mittel und Verfahren bekannt sind und in der Regel vorgesehen sind, die ein Zersetzen und einen Abbau von im Isolatorraum befindlichen Dekontaminationsmittel und/oder Dekontaminationsaerosol erlauben und erleichtern.

Die oben genannte Aufgabe wird auch durch eine Dekontaminationsanordnung, insbesondere für pharmatechnische Anwendungen, gelöst umfassend mindestens einen Isolatorraum und einen Dekontaminationsmittelvorratsbehälter aus dem wenigstens ein, bevorzugt mehrere, in dem Isolatorraum angeordnete oder an einer dem Isolatorraum begrenzenden Oberfläche angeordnete, bevorzugt als Zweistoffdüsen ausgebildete, Zerstäuber zum Erzeugen eines Dekontaminationsaerosols über mittels elektrische Steuermittel ansteuerbare Ventilmittel mit Dekontaminationsflüssigkeit, bevorzugt Wasserstoffperoxid, versorgbar sind, wobei die Zerstäuber über eine Gasleitung gasleitend mit einer Gasdruckquelle, insbesondere eine Druckluftquelle, zum Erzeugen eines Trägergasstroms für ein zu erzeugendes Dekontaminationsaerosol verbunden sind und wobei in der Gasleitung zwischen der Gasdruckquelle und den Zerstäubern, bevorzugt individuell für jeden Zerstäuber, ein Filter zur Filterung des Trägergasstroms angeordnet ist. Erfindungsgemäß ist weiter vorgesehen, dass der Abstand zwischen Filter und Zerstäuber in einer Gasleitung weniger als 5m beträgt und/oder dass die gasleitende Verbindung zwischen der Gasdruckquelle und den Zerstäubern einen, bevorzugt individuell für jeden Zerstäuber und/oder Filter vorgesehenen, Einleitungspunkt zur Einleitung von Dekontaminationsflüssigkeit umfasst.

Mit einer derartigen Dekontaminationsanordnung wird in vorteilhafter Weise erreicht, dass die vorangehend beschriebenen Dekontaminationsverfahren umgesetzt oder durchgeführt werden können. Durch den Einleitungspunkt in der Gasleitung kann entweder manuell, händisch oder teilautomatisiert/vollautomatisiert flüssiges Dekontaminationsmittel in die Gasleitung eingebracht werden.

Bei einem Einsaugen von Dekontaminationsaerosol über die Zerstäuber in die Gasleitung ist die dekontaminierbare Strecke der Gasleitung ab den Zerstäubern begrenzt. Mit anderen Worten ausgedrückt, nimmt die Dekontaminationszuverlässigkeit mit zunehmendem Abstand von dem Zerstäuber beim Einsaugen des Dekontaminationsaerosols in die Gasleitung ab. Auch beim Einbringen, insbesondere Einspritzen von flüssigem Dekontaminationsmittel durch einen Einleitungspunkt der Gasleitung kann besonders vorteilhaft eine beschränkte Distanz von weniger als 5m zwischen dem Zerstäuber und einem Filter die Dekontamination der Gasleitung, jenseits des Filters oder zwischen Filter und Zerstäuber besonders verbessern. Deshalb ist erfindungsgemäß vorgesehen oder kann erfindungsgemäß vorgesehen sein, dass zusätzlich oder alternativ zu dem Einleitungspunkt ein entsprechender Filter vorgesehen ist, der in einem Abstand von weniger als 5m gegenüber dem jeweiligen Zerstäuber angeordnet ist.

In einer ersten vorteilhaften Ausgestaltung der Dekontaminationsanordnung kann vorgesehen sein, dass wenigstens ein individueller Behälter für flüssiges Dekontaminationsmittel mit einer am Einleitungspunkt der Gasleitung vorgesehenen, verschließbaren Kupplung trennbar verbunden ist. Der individuelle Behälter kann dabei auch sukzessive oder nacheinander genutzt werden, um an unterschiedlichen Gasleitungen an entsprechenden verschließbaren Kupplungen angekoppelt oder angedockt zu werden, um jeweils Dekontaminationsflüssigkeit oder flüssiges Dekontaminationsmittel bereitzustellen und einzuleiten. Die verschließbare Kupplung kann bevorzugt so ausgestaltet sein, dass eine trennbare Verbindung mit dem Behälter möglich ist und gleichzeitig eine einfache Dekontamination der verschließbaren Kupplung selbst, insbesondere im Bereich der Verbindung mit dem Behälter, ermöglicht wird. Auch aufseiten des Behälters kann vorteilhaft vorgesehen sein, dass dieser so ausgestaltet ist, dass er im Bereich der Verbindung mit der verschließbaren Kupplung dekontaminierbar ist, beispielsweise händisch dekontaminierbar ist.

In einer weiteren, vorteilhaften Ausführungsform der Dekontaminationsanordnung kann vorgesehen sein, dass der Einleitungspunkt über eine mittelbare Verbindung mit dem Dekontaminationsmittelvorratsbehälter verbunden ist. Die Verbindung kann beispielsweise als weitere Schlauchleitung ausgebildet sein. Auch kann ein entsprechend verzweigtes System von Schlauchleitungen vorgesehen sein, um flüssiges Dekontaminationsmittel aus dem Dekontaminationsmittelvorratsbehälter zum Einleitungspunkt der jeweiligen Gasleitung und von dort aus in die Gasleitung einzubringen. Durch diese wenigstens mittelbaren Verbindungen, insbesondere Leitungen, kann eine teilautomatisierte oder vollautomatisierte Einbringung des flüssigen Dekontaminationsmittels in die Gasleitung erreicht werden.

In einer weiteren, vorteilhaften Ausgestaltung der Dekontaminationsanordnung kann außerdem vorgesehen sein, dass die Dekontaminationsanordnung eine Unterdruckquelle umfasst, die fluidleitend mit einer Gasleitung oder einem Teil einer Gasleitung verbunden ist. Über die Gasleitung kann, bevorzugt ausgehend von einer Gasdruckquelle ein Trägergasstrom an dem Zerstäuber bereitgestellt werden.

Bevorzugt ist eine fluidleitende Verbindung der Unterdruckquelle, bevorzugt einer Unterdruckpumpe oder Vakuumpumpe, mit der Fluidleitung schaltbar ausgebildet. Dies kann vorteilhaft durch entsprechende Ventile realisiert werden. In einer ersten Ventil- oder Schaltstellung ist der Zugang oder die Verbindung zwischen Gasleitung und der Fluidleitung zur Unterdruckquelle geschlossen und gleichzeitig die Fluidleitung zwischen einer Gasdruckquelle und einem Zerstäuber geöffnet. In diesem Zustand kann der Trägergasstrom am Zerstäuber ausgegeben werden und dort Dekontaminationsmittel zerstäuben. Gleichzeitig kann der Trägergasstrom nicht von der Unterdruckquelle angesaugt oder aus der Gasleitung ausgesaugt werden. In einer zweiten Ventil- oder Schaltstellung ist die Fluidleitung zwischen Gasdruckquelle und Zerstäuber unterbrochen und stattdessen die Fluidleitung zwischen Unterdruckquelle und Zerstäuber geöffnet. Der an der Schaltstelle oder Ventil anliegende Trägergasstrom kann nicht zum Zerstäuber gelangen. Stattdessen kann ein Unterdruck in einem Teil der Gasleitung ausgebildet werden, der am Zerstäuber dafür sorgt, dass aus dem Isolatorraum Gas oder Aerosol angesaugt wird.

Damit wird in besonders vorteilhafter Weise das Einbringen eines Dekontaminationsaerosols vom Isolatorraum über die Zerstäuber in die Gasleitung erreicht und ermöglicht. Durch die schaltbare Verbindung der Zerstäuber mit der Gasdruckquelle und der Unterdruckquelle kann vorteilhaft eine einzelne oder gruppenweise Umschaltung von einzelnen Gasleitungen und Zerstäubern so erfolgen, dass ein Teil der Zerstäuber weiterhin Dekontaminationsmittel ausgibt oder zerstäubt, während andere Zerstäuber das in den Isolatorraum abgegebene Aerosol einsaugen.

Zwischen der schaltbaren Verbindung der Gasleitung mit der Gasdruckquelle und der Unterdruckquelle und dem Zerstäuber kann vorteilhaft ein Filter angeordnet sein. Dadurch wird sichergestellt, dass beim Einsaugen von Dekontaminationsaerosol aus dem Isolatrorraum die Gasleitung von dem Zerstäuber bis wenigstens zu dem Filter dekontaminiert wird.

Vorteilhafte Ausgestaltungen der vorliegenden Erfindung werden nachfolgend anhand der beispielhaften, rein schematischen Zeichnungen erläutert.

Darin zeigen:
- Fig. 1a:: eine schematische Darstellung eines Ausschnitts einer erfindungsgemäßen Dekontaminationsanordnung in einer ersten Ausführungsform;
- Fig. 1b:: eine schematische Darstellung eines Ausschnitts einer erfindungsgemäßen Dekontaminationsanordnung in einer zweiten Ausführungsform
- Fig. 2:: einen schematischen Ausschnitt aus einer erfindungsgemäßen Dekontaminationsanordnung in einer zweiten Ausführungsform;
- Fig. 3:: ein Ablaufdiagramm für ein erfindungsgemäßes Dekontaminationsverfahren in einer ersten Ausführungsform;
- Fig. 4:: ein Ablaufdiagramm für ein erfindungsgemäßes Dekontaminationsverfahren nach einer zweiten Ausführungsform;
- Fig. 5:: ein Ablaufdiagramm für ein erfindungsgemäßes Dekontaminationsverfahren gemäß einer dritten Ausführungsform.

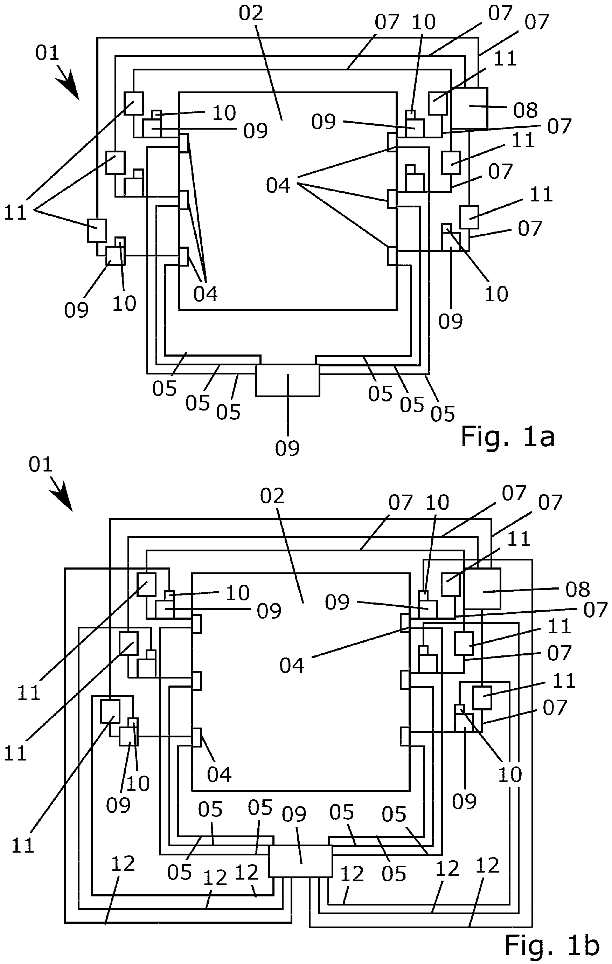

Die Fig. 1 zeigt einen Ausschnitt aus einer Dekontaminationsanordnung 01. Dargestellt ist ein Teil eines Isolatorraums 02 an dessen Grenzen, insbesondere in dessen Wänden 03 mit entsprechenden Öffnungen zum Islatorraum 02 eine Vielzahl von Zerstäubern 04 angeordnet sind. Die Zerstäuber 04 sind beispielsweise als Zweistoffdüsen ausgebildet und sind dementsprechend einerseits mit einer Dekontaminationsmittelleitung 05 mit einem Dekontaminationsmittelvorratsbehälter 06 verbunden. Weiterhin sind die Zerstäuber 04 über Gasleitungen 07 mit einer Gasdruckquelle 08 verbunden. Die Gasdruckquelle 08 dient dazu im Dekontaminationsverfahren des Isolatorraums 02 ein Trägergasstrom durch die Gasleitungen 07 an den Zerstäubern 04 zur Verfügung zu stellen, der ein über die Zerstäuber 04 ausgebrachtes oder ausgetragenes Dekontaminationsmittel, beispielsweise Wasserstoffperoxid (H₂O₂) zerstäubt und in ein Dekontaminationsaerosol überführt.

In der Ausführungsform der Fig. 1 sind in den Gasleitungen 07 Einleitungspunkte 09 vorgesehen. An den Einleitungspunkten 09 können verschließbare Kupplungen 10 ausgebildet sein. Über die verschließbaren Kupplungen 10 kann über eine lösbare Verbindung mit einem Behälter, insbesondere einem Dekontaminationsmittelbehälter, flüssiges Dekontaminationsmittel in die Gasleitungen 07 eingebracht werden, welches dann über einen von der Gasdruckquelle 08 erzeugten und geförderten Trägergasstrom durch die Gasleitungen 07 und von den Einleitungspunkten 09 zu den Zerstäubern 04 und von dort aus in den Isolatorraum 02 befördert werden kann. Dadurch kann die Gasleitung 07 ab dem Einleitungspunkt 09 dekontaminiert werden. Um eine Auswirkung einer Dekontamination des Trägergasstroms oder eine Auswirkung einer Dekontamination der Gasleitung 07 zwischen den Einleitungspunkten 09 und der Gasdruckquelle 08 zu verhindern sind zwischen den Einleitungspunkten 09 und der Gasdruckquelle 08, möglichst direkt angrenzend oder benachbart zu den Einleitungspunkten 09 Filter 11 vorgesehen, die den von der Gasdruckquelle 08 gelieferten Trägergasstrom filtern.

In der abgewandelten Darstellung der Fig. 1b ist die grundsätzliche Struktur der Dekontaminationsanordnung 01 ähnlich, wie in der Fig. 1a. Gleiche Bezugszeichen in der Fig. 1b weisen auf Komponenten mit gleicher Funktion hin. Es ist erkennbar, dass in der Fig. 1b anstatt der verschließbaren Kupplungen 10 eine weitere, wenigstens mittelbare Verbindung 12 zwischen den Einleitungspunkten 09 der Gasleitungen 07 und dem Dekontaminationsmittelvorratsbehälter 06 ausgebildet ist. Damit kann, beispielsweise über nicht im Detail dargestellte Fördermittel, beispielsweise Pumpen, flüssiges Dekontaminationsmittel direkt aus dem Dekontaminationsmittelvorratsbehälter 06 entnommen werden und an dem Einleitungspunkt 06 oder den Einleitungspunkten 06 in die Gasleitung 07 eingebracht werden. Auch bei dieser Ausgestaltung kann es besonders vorteilhaft vorgesehen sein, dass das eingebrachte, flüssige Dekontaminationsmittel über einen entsprechenden Trägergasstrom, wie bereits mit Bezug zur Fig. 1a beschrieben, aus den Gasleitungen 07 über die Zerstäuber 04 in den Isolatorraum 02 abgegeben oder ausgetragen wird.

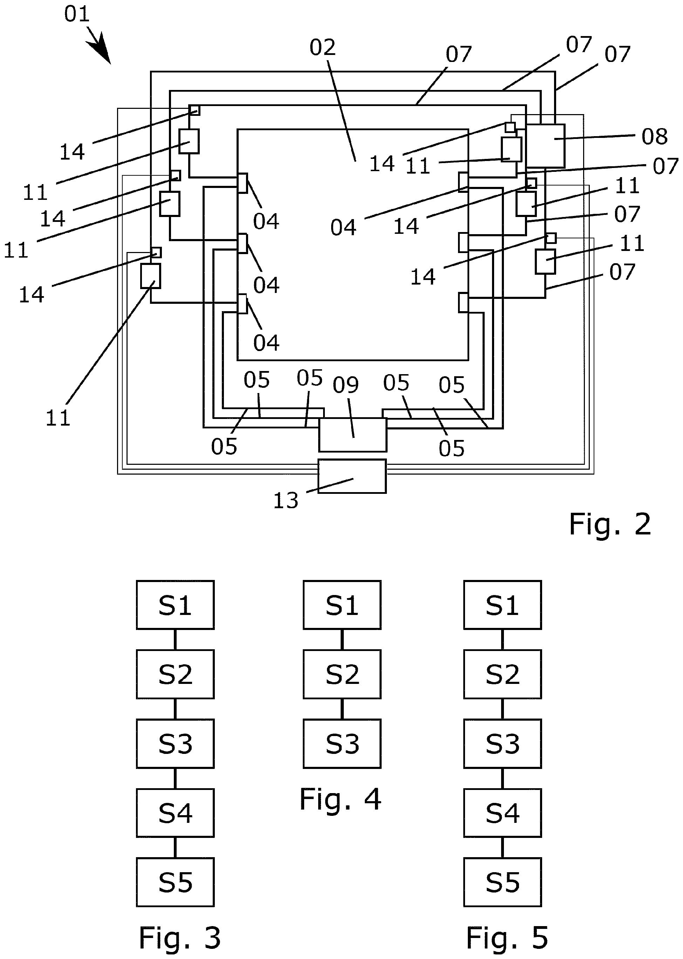

In der Ausgestaltung der Fig. 2 kann auf die Einleitungspunkte 09, die verschließbaren Kupplungen 10 und die zusätzlichen, mittelbaren Verbindungen 12 mit dem Dekontaminationsmittelvorratsbehälter 06 verzichtet werden. Stattdessen ist in dieser Ausführungsform eine schaltbare Verbindung, bevorzugt ein Ventil 14, in der Gasleitung angeordnet, die eine schaltbare fluidleitende Verbindung der Zerstäuber 04 mit der Gasdruckquelle 08 oder der Unterdruckquelle 13 ermöglicht. Dadurch kann die Gasleitung 07 im Bereich zwischen den Zerstäubern 04 und dem Ventil 14 entweder mit dem Trägergasstrom der Gasdruckquelle 08 oder einem Unterdruck der Unterdruckquelle 13 beaufschlagt werden.

Über eine entsprechende Regelung der des Ventils 14 kann der Überdruck oder der Unterdruck und damit auch die Flussrate beeinflusst werden. Wie nachfolgend noch im Detail beschrieben werden wird, wird bei der Ausführungsform der Fig. 2 im Isolatorraum 02 ein Dekontaminationsaerosol hergestellt, welches dann über eine entsprechende Beaufschlagung der Gasleitungen 07, bevorzugt eines Teils oder eine Gruppe der Gasleitungen 07, mit Unterdruck und eine damit einhergehenden Erzeugung eines Rückgasstroms aus dem Isolatorraum 02 über die Zerstäuber 04 durch die Gasleitungen 07 in Richtung der Unterdruckquelle 13 auch in die Gasleitung 07 eingeführt, insbesondere eingesogen, wird. Das eingesogene Dekontaminationsaerosol kann, sofern die Konzentration an Dekontaminationsmittel im Isolatorraum 02 ausreichend hoch ist und ausreichend hoch bleibt und der Unterdruck in den Gasleitungen 07 nicht zu groß ist dazu benutzt werden, um die Gasleitungen 07, mindestens bis zu den Filtern 11 zu dekontaminieren. Bevorzugt wird über einen Teil der Zerstäuber 04 und den angeschlossenen Gasleitungen 07 Trägergasstrom ausgegeben und Dekontaminationsmittel zerstäubt, während über einen Zerstäuber oder eine Gruppe von Zerstäuber Dekontaminationsaerosol aus dem Isolatorraum 02 eingesaugt wird.

In dem Ablaufdiagramm der Fig. 3 wird ein Verfahren dargestellt, welches bevorzugt unter Verwendung einer Dekontaminationsanordnung gemäß der Fig. 1a durchgeführt wird. In einem ersten Verfahrensschritt S1 kann vorgesehen sein, dass zunächst die Filter 11 ausgetauscht und durch neue, sterile oder vordekontaminierte Filter 11 ersetzt werden. In einem nachfolgenden Verfahrensschritt S3 kann dann vorteilhaft vorgesehen sein, dass individuell oder gleichzeitig an einzelnen oder allen verschließbaren Kupplungen 10 entsprechende Behälter mit flüssigem Dekontaminationsmittel zeitweise angekoppelt werden. In einem vorausgehenden Verfahrensschritt S2 kann vorteilhaft vorgesehen sein, dass die verschließbaren Kupplungen 10 und/oder die Behälter zunächst selbst an den entsprechenden Kontaktflächen oder Kontaktstellen gereinigt und/oder dekontaminiert werden.

Im nachfolgenden Verfahrensschritt S4 kann vorteilhaft vorgesehen sein, dass das flüssige Dekontaminationsmittel aus den Behältern über die verschließbaren Kupplungen die Einleitungspunkte 09 und damit in die Gasleitung 07 gelangen. Das Entleeren der Behälter kann entweder händisch, beispielsweise in Form der Betätigung eines Kolbens, automatisch, beispielsweise durch Ausbildung der Behälter als Patrone, die neben oder zusätzlich zum flüssigen Dekontaminationsmittel auch ein Treibmittel oder Treibgas aufweisen oder physikalisch, beispielsweise durch die Wirkung des Trägergasstroms auf das Dekontaminationsmittel im Behälter im Sinne einer Strahldüse oder einer Venturidüse erreicht werden. Auch ein Einbringen der Dekontaminationsflüssigkeit oder des flüssigen Dekontaminationsmittels aufgrund oder unter Zuhilfenahme von Schwerkraft/Gewichtskraft kann vorteilhaft sein.

Nachdem die Behälter entleert und das Dekontaminationsmittel, insbesondere das flüssige Dekontaminationsmittel von den Einleitungspunkten 09 entlang der Gasleitungen 07 vom Trägergasstrom bis zu den Zerstäubern 04 und von dort aus in den Isolatorraum 02 befördert wurde, können die Behälter in einem weiteren Verfahrensschritt S5 von der verschließbaren Kupplung gelöst und die verschließbare Kupplung - gegebenenfalls nach einer nochmaligen Reinigung/Dekontamination - verschlossen werden.

Eine leicht abgewandelte Verfahrensführung, die eine Dekontaminationsanordnung gemäß der Fig. 1b verwendet ist in der Darstellung der Fig. 4 wiedergegeben. Hier kann in einem ersten Verfahrensschritt S1 abermals der Tausch von gebrauchten oder benutzten Filtern 11 gegen neue und bevorzugt vorsterilisierte oder dekontaminierte Filter 11 erfolgen. In einem nachfolgenden Verfahrensschritt S2 wird automatisch oder teilautomatisiert Dekontaminationsmittel, insbesondere flüssiges Dekontaminationsmittel über die Verbindungen 12 aus dem Dekontaminationsmittelvorratsbehälter 06 entnommen und an den Einleitungspunkten 09 der Gasleitungen 07 zur Verfügung gestellt. In einem anschließenden Verfahrensschritt S3 kann, bevorzugt solange flüssiges Dekontaminationsmittel nachgeliefert wird über einen Trägergasstrom von der Gasdruckquelle 08 in Richtung der Zerstäuber 04 das flüssige Dekontaminationsmittel von den Einleitungspunkten 09 durch die Gasleitungen 07 und über die Zerstäuber 04 in den Isolatorraum 02 ausgetragen werden.

Eine alternative Verfahrensführung, bevorzugt unter Verwendung einer Dekontaminationsanordnung gemäß der Fig. 2 ist in der Fig. 5 dargestellt. Hier kann vorteilhaft in einem ersten Verfahrensschritt S1 auch ein, wie vorangehend bereits beschriebener, Filterwechsel erfolgen. In einem Verfahrensschritt S2 kann dann mit allen Zerstäubern 04 zunächst eine gewünschte Konzentration von Dekontaminationsmittel in Form von Dekontaminationsaerosol im Isolatorraum 02 hergestellt werden, in dem über die Dekontaminationsmittelleitungen 05 Dekontaminationsmittel, beispielsweise Wasserstoffperoxid an die Zerstäuber 04 geführt wird und durch einen Trägergasstrom, der durch die Gasleitungen 07 vonseiten der Gasdruckquelle 08 bereitgestellt wird, zerstäubt werden.

In einem nachfolgenden Verfahrensschritt S3 kann dann vorgesehen sein, dass einzelne Zerstäuber 04 oder Gruppen von Zerstäubern 04 Dekontaminationsaerosol aus dem Isolatorraum 02 entnehmen, insbesondere absaugen und durch die Gasleitungen 07 in Richtung der Unterdruckquelle 13 transportieren. Dazu kann eine entsprechende Umschaltung des Ventils 14 erfolgen, so dass zumindest in einzelnen oder einigen Gasleitungen 07 einen entsprechenden Unterdruck generiert und die Verbindung zum Trägergasstrom der Gasdruckquelle 08 unterbrochen wird . Währenddessen kann voreilhaft vorgesehen sein, dass andere Zerstäuber 04 unter Zulieferung von Trägergasstrom und Dekontaminationsmittel weiterhin Dekontaminationsaerosol erzeugen und in den Isolatorraum 02 abgeben, um ein zu schnelles oder zu starkes Absinken der Konzentration des Dekontaminationsmittels im Isolatorraum 02 zu verhindern. Für die letztgenannte Gruppe von Zerstäubern 04 kann das Ventil 14 so geschaltet sein, dass der Unterdruck der Unterdruckquelle 13 nicht auf die Gasleitung 07 wirken kann und stattdessen der Trägergasstrom auf Grund eines von der Gasdruckquelle verursachten Überdrucks an den Zerstäubern anliegt und von dieses ausgegeben wird.

In einem weiteren Verfahrensschritt S4 oder in weiteren Verfahrensschritten S4.1 bis S4.N können einzeln oder gruppenweise weitere Zerstäuber 04 ein Einsaugen von Dekontaminationsaerosol aus dem Isolatorraum 02 bewirken, während bevorzugt andere Zerstäuber 04, wie vorangehend bereits beschrieben, weiterhin Dekontaminationsaerosol in den Isolatorraum 02 abgeben. Dazu können gezielt die Ventile 14 geschaltet werden.

In einem nachfolgenden Verfahrensschritt S5 können die evtl. in den Gasleitungen 07 verbliebenen Reste von Dekontaminationsmittel und/oder Dekontaminationsaerosol für die jeweiligen Gasleitungen 07 einzeln, gruppenweise oder gleichzeitig entleert werden, indem abermals die Ventile 14 umgeschaltet und ein Trägergasstrom ausgehend von der Gasdruckquelle 08 in Richtung der Zerstäuber 04 so erzeugt wird, dass Reste über die Gasleitungen 07 und die Zerstäuber 04 ebenfalls in den Isolatorraum 02 befördert oder ausgeblasen werden.

### Bezugszeichen:

- 01: Dekontaminationsanordnung
- 02: Isolatorraum
- 03: Wände
- 04: Zerstäuber
- 05: Dekontaminationsmittelleitung
- 06: Dekontam inationsm ittelvorratsbehälter
- 07: Gasleitungen
- 08: Gasdruckquelle
- 09: Einleitungspunkte
- 10: verschließbare Kupplung
- 11: Filter
- 12: Verbindung
- 13: Unterdruckquelle
- 14: Ventil

- S1: Verfahrensschritt
- S2: Verfahrensschritt
- S3: Verfahrensschritt
- S4: Verfahrensschritt
- S5: Verfahrensschritt

## Patentansprüche

1. Dekontaminationsverfahren, insbesondere für pharmatechnische Anwendungen,
**gekennzeichnet durch** die Verfahrensschritte:
- Einbringen von einem Dekontaminationsmittel in wenigstens eine Gasleitung (07), die zur Versorgung von wenigstens einem, in einem Isolatorraum (02) angeordneten, bevorzugt als Zweistoffdüse ausgebildeten, Zerstäuber (04) mit einem Trägergasstroms, bevorzugt Druckluft, wobei zum Erzeugen eines Dekontaminationsaerosols in einem Dekontaminationsbetrieb des Isolatorraums (02) der wenigstens eine Zerstäuber (04) über mittels elektronische Steuermittel ansteuerbare Ventilmittel mit Dekontaminationsflüssigkeit, bevorzugt Wasserstoffperoxid, aus einem Dekontaminationsmittelvorratsbehälter (06) versorgt wird,
wobei der wenigstens eine Zerstäuber (04) gasleitend mit einer Gasdruckquelle (08), über die Gasleitung (07) verbunden ist und in der Gasleitung (07) zwischen der Gasdruckquelle (08) und den Zerstäubern (04), bevorzugt individuell für jeden Zerstäuber (04), ein Filter (11) zur Filterung des Trägergasstroms angeordnet ist.

2. Dekontaminationsverfahren nach Anspruch 1,
**dadurch gekennzeichnet,**
**dass** flüssiges Dekontaminationsmittel in die Gasleitung (07) eingebracht wird und mit dem Trägergasstrom über den Zerstäuber (04) in den Isolatorraum (02) ausgetragen wird.

3. Dekontaminationsverfahren nach Anspruch 2,
**dadurch gekennzeichnet,**
**dass** das flüssige Dekontaminationsmittel an einem, bevorzugt für jeden Zerstäuber (04) individuellen, Einleitungspunkt (09) in der Gasleitung (07) eingebracht wird.

4. Dekontaminationsverfahren nach Anspruch 2 oder 3,
**dadurch gekennzeichnet,**
**dass** das flüssige Dekontaminationsmittel an einem Einleitungspunkt (09) eingebracht wird, der in der Gasleitung (07) zwischen einem Filter (11) und dem Zerstäuber (04) angeordnet ist.

5. Dekontaminationsverfahren nach einem der Ansprüche 2 bis 4,
**dadurch gekennzeichnet,**
**dass** zur Dekontamination der Gasleitung (07) ein Behälter mit einem, bevorzugt vordosiertes, Volumen an flüssigem Dekontaminationsmittel während einer zeitweisen Verbindung (12) an einer verschließbaren Kupplung (10) der Gasleitung (07) am Einleitungspunkt (09) angekoppelt und entleert wird.

6. Dekontaminationsverfahren nach einem der Ansprüche 2 bis 5,
**dadurch gekennzeichnet,**
**dass** zur Dekontamination der Gasleitung (07) das flüssige Dekontaminationsmittel über eine permanente Zuleitung bereitgestellt wird, die über eine wenigstens mittelbare Verbindung (12) zwischen dem Dekontaminationsmittelvorratsbehälter (06) und dem Einleitungspunkt (09), bevorzugt mittels entsprechender Fördermittel, Dekontaminationsmittel aus dem Dekontaminationsflüssigkeitsbehälter (06) in die Gasleitung (07) fördert.

7. Dekontaminationsverfahren nach Anspruch 1,
**dadurch gekennzeichnet,**
**dass** Dekontaminationsmittel in der Form eines Dekontaminationsaerosols in die Gasleitung (07) eingebracht wird.

8. Dekontaminationsverfahren nach Anspruch 7,
**dadurch gekennzeichnet,**
**dass** das Dekontaminationsaerosol aus dem Isolatorraum (02) in die Gasleitung (07) eingebracht wird und durch einen Trägergasstrom in die Gasleitung (07) eingebracht, insbesondere eingesaugt, wird, der eine umgekehrte Flussrichtung aufweist, als der Trägergasstrom während des Dekontaminationsbetriebs des Isolatorraums (02).

9. Dekontaminationsverfahren nach Anspruch 7 oder 8,
**dadurch gekennzeichnet,**
**dass** ein erster Teil einer Gesamtheit von mehreren Zerstäubern (04) ein Dekontaminationsaerosol in den Isolatorraum (02) zerstäubt während ein zweiter Teil der Gesamtheit der Zerstäuber (04) das Dekontaminationsaerosol aus dem Isolatorraum (02) entnimmt und insbesondere in die Gasleitung (07) des zweiten Teils der Gesamtheit der Zerstäuber (04) einbringt, insbesondere einsaugt.

10. Dekontaminationsverfahren nach einem der Ansprüche 7 bis 9,
**dadurch gekennzeichnet,**
**dass** vor dem Einbringen des Dekontaminationsaerosols in die Gasleitung (07) die Gesamtheit der Zerstäuber (04) ein Dekontaminationsaerosol in den Isolatorraum (02) zerstäubt.

11. Dekontaminationsverfahren nach einem der Ansprüche 7 bis 10,
**dadurch gekennzeichnet,**
**dass** nach dem Einbringen des Dekontaminationsaerosols in die Gasleitung (07) die Flussrichtung des Trägergasstroms umgekehrt und in der Gasleitung (07) verbliebenes, bevorzugt flüssiges, Dekontaminationsmittel und/oder Dekontaminationsaerosol mittels des Trägergasstroms über die Zerstäuber (04) in den Isolatorraum (02) ausgetragen wird.

12. Dekontaminationsanordnung (01), insbesondere für pharmatechnische Anwendungen, bevorzugt zur Ausführung des Dekontaminationsverfahrens nach einem der Ansprüche 1 bis 11, umfassend mindestens einen Isolatorraum (02) und
einen Dekontaminationsmittelvorratsbehälter (06) aus dem wenigstens ein, bevorzugt mehrere, in dem Isolatorraum (02) angeordnete, bevorzugt als Zweistoffdüsen ausgebildete, Zerstäuber (04) zum Erzeugen eines Dekontaminationsaerosols über mittels elektronische Steuermittel ansteuerbare Ventilmittel mit Dekontaminationsflüssigkeit, bevorzugt Wasserstoffperoxid, versorgbar sind,
wobei die Zerstäuber (04) über eine Gasleitung (07) gasleitend mit einer Gasdruckquelle (08), insbesondere einer Druckluftquelle, zum Erzeugen eines Trägergasstroms für ein zu erzeugendes Dekontaminationsaerosols verbunden sind und wobei in der Gasleitung (07) zwischen der Gasdruckquelle (08) und den Zerstäubern, (04) bevorzugt individuell für jeden Zerstäuber (04), ein Filter (11) zur Filterung des Trägergasstroms angeordnet ist,
**dadurch gekennzeichnet,**
**dass** der Abstand zwischen Filter (11) und Zerstäuber (04) in einer Gasleitung (07) weniger als 5m beträgt und/oder dass die gasleitende Verbindung (12) zwischen der Gasdruckquelle (08) und den Zerstäubern (04) einen, bevorzugt individuell für jeden Zerstäuber (04) und/oder Filter (11) vorgesehenen, Einleitungspunkt (09) zur Einleitung von Dekontaminationsflüssigkeit umfasst.

13. Dekontaminationsanordnung nach Anspruch 12,
**dadurch gekennzeichnet,**
**dass** wenigstens ein individueller Behälter für flüssiges Dekontaminationsmittel mit einer am Einleitungspunkt (09) vorgesehenen, verschließbaren Kupplung (10) trennbar verbunden ist.

14. Dekontaminationsanordnung nach Anspruch 12,
**dadurch gekennzeichnet,**
**dass** der Einleitungspunkt (09) über eine wenigstens mittelbare Verbindung (12) mit dem Dekontaminationsmittelvorratsbehälter (06) verbunden ist.

15. Dekontaminationsanordnung nach Anspruch 12,
**dadurch gekennzeichnet,**
**dass** die die Dekontaminationsanordnung eine Unterdruckquelle (13) umfasst, die fluidleitend, insbesondere schaltbar, mit einer Gasleitung (07) oder einem Teil einer Gasleitung (07) verbunden ist.
